(19) Europäisches Patentamt
European Patent Office
Office européen des brevets

(11) Veröffentlichungsnummer: **0 008 368**
**B1**

(12) # EUROPÄISCHE PATENTSCHRIFT

(21) Anmeldenummer: **79102525.7**

(51) Int. Cl.³: **C 07 D 285/16**

(22) Anmeldetag: **18.07.79**

(54) **Verfahren zur Herstellung von Cyanoverbindungen**

(30) Priorität: **24.07.78 DE 2832404**

(43) Veröffentlichungstag der Anmeldung:
**05.03.80 Patentblatt 80/05**

(45) Bekanntmachung des Hinweises auf die Patenterteilung:
**12.11.80 Patentblatt 80/23**

(84) Benannte Vertragsstaaten:
**AT BE CH DE FR GB IT LU NL SE**

(56) Entgegenhaltungen:
**DE - A1 - 2 444 822**
**DE - A1 - 2 656 289**

(73) Patentinhaber: **BASF Aktiengesellschaft**
**Carl - Bosch - Strasse 38**
**D - 6700 Ludwigshafen (DE)**

(72) Erfinder: **Linhart, Friedrich, Dr. Chem.**
**Leisberg 61**
**D - 6900 Heidelberg (DE)**
**Stubenrauch, Gerd, Dr. Chem.**
**Rubensstrasse 36**
**D - 6700 Ludwigshafen (DE)**
**Hamprecht, Gerhard, Dr. Chem.**
**Rote - Turm - Strasse 28**
**D - 6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 368 B1

Verfahren zur Herstellung von Cyanoverbindungen

Die vorliegende Erfindung betrifft ein Verfahren zur Herstellung von 1-Cyano-2,1,3-benzothiadiazinonverbindungen der allgemeinen Formel I

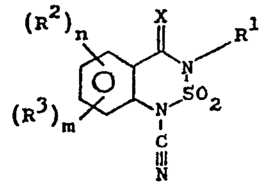

I,

worin

$R^1$ Alkyl, Halogenalkyl, Cycloalkyl, Alkenyl, Alkinyl, Halogenalkenyl, Halogenalkinyl, Alkoxyalkyl, Alkylmercaptoalkyl, Alkyl- und Dialkylcarbamoylalkyl, Alkoxycarbonyl, Alkoxycarboalkyl, Alkoxycarboalkenyl, einen Alkanoylalkyl-, einen gegebenenfalls halogen-, methyl- oder halogen-methylsubstituierten Arylrest oder einen heterocylischen Ring,

$R^2$ und

$R^3$ unabhängig voneinander Halogen, Nitro, Niedrigalkyl, Alkoxyalkyl, Halogenniedrigalkyl, Cycloalkyl, Arylalkyl, Aryl, CN, SCN, $CO_2R^4$, $C(=O)N\genfrac{}{}{0pt}{}{R^4}{R^5}$, $N\genfrac{}{}{0pt}{}{R^4}{R^5}$, $YR^5$, $SO_2R^4$, $SO_2OR^4$, $SO_2N\genfrac{}{}{0pt}{}{R^4}{R^5}$, $(C{=}O)R^4$ oder $Y'CF_2C(Z)_3$,

$R^4$ und

$R^5$ unabhängig voneinander gegebenenfalls halogen-, methyl- oder nitro-substituiertes Niedrigalkyl oder Aryl bedeuten, jedes

X,Y und

Y' unabhängig Sauerstoff oder Schwefel, jedes

Z unabhängig Wasserstoff oder Halogen und

n und

m unabhängig voneinander ganze Zahlen von 0 bis 4 bedeuten mit der Maßgabe, daß m + n nicht größer als 4 ist durch Umsetzung einer wäßrigen Lösung eines Salzes der Verbindung I mit einem anorganischen Cyanid und Brom.

Die Bezeichnung "Halogen" bedeutet Fluor, Chlor, Brom, Jod.

Die Bezeichnung "Niedrigalkyl" und "Halogenniedrigalkyl" bedeutet verzweigtes oder geradkettiges, gegebenenfalls halogensubstituiertes Alkyl mit 1 bis 6 Kohlenstoffatomen.

Die Bezeichnung "Cycloalkyl" bedeutet beispielsweise Cyclopropyl, Cyclobutyl, Cyclopentyl, Cyclohexyl, Cycloheptyl sowie alkylsubstituierte Cycloalkylreste.

Die Bezeichnung "Aryl" bedeutet Phenyl oder substituiertes Phenyl z.B. Halogenphenyl oder Tolyl.

$R^1$ kann u.a. bedeuten: Wasserstoff, Methyl, Äthyl, n-Propyl, Isopropyl, Cyclopropyl, n - Butyl, Isobutyl, tert. - Butyl, Cyclobutyl, n - Pentyl, 2 - Pentyl, 3 - Pentyl, tert. - Amyl, Neopentyl, 2 - Methylbutyl, 3 - Methyl - butyl, 3 - Methyl - 2 - butyl, Cyclopentyl, n - Hexyl, 4 - Methyl - 2 - pentyl, 2,3 - Dimethylbutyl, 2 - Methyl - 1 - pentyl, 2 - Hexyl, 3 - Hexyl, 3 - Methyl - 2 - pentyl, 3 - Methylpentyl, 4 - Methylpentyl, 3 - Methyl - 3 - pentyl, 4,4 - Dimethylbutyl, Cyclohexyl, Heptyl, 2 - Heptyl, 3 - Heptyl, 4 - Heptyl, Cycloheptyl, 1 - Octyl, 2 - Octyl, 3 - Octyl, 4 - Octyl, 5 - Octyl, 5 - Äthyl - 2 - heptyl, 2,6 - Dimethyl - 4 - heptyl, 7 - Äthyl - 2 - methyl - 4 - nonyl, 2,4 - Dimethyl - 3 - pentyl, 3 - Methyl - 2 - heptyl, 5 - Äthyl - 2 - nonyl, Nonyl, Decyl, Undecyl, Dodecyl, Tridecyl, Tetradecyl, Pentadecyl, Hexadecyl, Heptadecyl, Octadecyl, Nonadecyl, Eicosyl, 6 - Äthyl - 3 - decyl, 6 - Äthyl - 3 - octyl, 2 - Methyl - 2 - pentyl, 2,3 - Dimethyl - 2 - butyl, 2 - Methyl - 2 - hexyl, 3 - Äthyl - 3 - pentyl, 3 - Methyl - 3 - hexyl, 2,3 - Dimethyl - pentyl - 3, 2,4 - Dimethyl - 2 - pentyl, 2,2,3 - Trimethyl - 3 - butyl, 2 - Methyl - 2 - heptyl, 4 - Methyl - 4 - heptyl, 2,4 - Dimethyl - 2 - hexyl, 2 - Methyl - 2 - octyl, 1 - Methyl - 1 - cyclopentyl, 1 - Methyl - 1 - cyclopentyl, 1 - Methyl - 1 - cyclohexyl, 1 - Äthyl - 1 - cyclohexyl, Chlor - tert. - butyl, 1,1 - Dichlor - 2 - methyl - 2 - propyl, 1,3 - Dichlor - 2 - methyl - 2 - propyl, 1 - Cyclohexyl - 1 - äthyl, 1 - Chloräthyl, 2 - Chloräthyl, 1 - Chlorpropyl, 2 - Chlorpropyl, 3 - Chlorpropyl, 1 - Chlor - 2 - propyl, 2 - Chlorbutyl, 2 - Chlor - 2 - methyl - 3 - propyl, 1 - Fluoräthyl, 2 - Fluoräthyl, 2 -Fluorpropyl, 3 - Fluorpropyl, 1 - Fluor - 2 - propyl, 2 - Fluorbutyl, 2 - Fluor - 2 - methyl - 3 - propyl, 2 - Bromäthyl, 3 - Brompropyl, 4 - Chlorbutyl, 2 - Chlorcyclohexyl, 1,1,1 - Trifluorisopropyl, Hexafluor - 2 - methyl - isopropyl, Hexachlorisopropyl, 1,2 - Dibrom - Allyl, 2,2,2 - Trifluoräthyl, 1 - Chlor - butin - 2 - yl - 4, 3 - Chlor - butin - 1 - yl - 4, 1 - Chlor - buten - 2 - yl - 4, 2,3 - Dibrom - 1 - propyl, 2,2,2 - Trichloräthyl, 1 - Chlorpentin - 2 - yl - 4, 2,2,2 - Tribromäthyl, 3,4,4 - Trichlorbuten - 3 - yl - 2, 1 - Brom - 2 - propyl, 1,3 - Dibrom - 2 -

propyl, 3 - Chlorbuten - 1 - yl - 4, Allyl, Methallyl, Crotyl, 2 - Athylhexen - 2 - yl - 1, Hexen - 5 - yl - 1, Hexen - 5 - yl - 1, Undecen - 10 - yl - 1, 2 - Methyl - buten - 2 - yl - 1, 2 - Methylbuten - 1 - yl - 3, Butin - 1 - yl - 3, Butin - 2 - yl - 1, Buten - 1 - yl - 3, Propargyl, 2 - Methylbuten - 1 - yl - 4, 2 - Methyl - buten - 2 - yl - 4, 3 - Methyl - buten - 1 - yl - 3, 1 - Äthinylcyclohexyl, Methoxyäthyl, Äthoxyäthyl, 3 - Methoxypropyl, Methoxyisopropyl, 3 - Methoxybutyl, 1 - Methoxy - butyl - 2, Äthoxy - tert. - butyl, Methoxy - tert. - butyl, Cyclohexoxy - tert. - butyl, 2 - Methoxy - butyl, 4 - Methoxy - butyl, Methyl - mercapto - äthyl, Äthylmercaptoäthyl, 3 - Methyl - mercapto - propyl, 3 - Methyl - mercaptobutyl, 1 - Methylmercapto butyl - 2, Methylmercapto - tert. - butyl, 2 - Methylmercaptobutyl, 4 - Methylmercaptobutyl, 3 - n - Butoxyäthyl, 2 - Äthoxypropyl, 3 - Äthoxy - 2 - propyl, 2 - Methyl - butanon - 3 - yl - 2, 2 - Methylpentanon - 4 - yl - 2, 3 - Butanon - 1 - yl, 3 - Butanon - 2 - yl, 2 - Propanon - 1 - yl, 2 - Pentanon - 1 - yl, Methylacetat - 2, Äthylacetat - 2, Methyl - propionat - 2, Methylpropionat - 3, Methylbutyrat - 2, Methylbutyrat - 3, Methylbutyrat - 4, Methyl - (2 - vinylpropionat - 2), Methyl - (2 - vinylacetat - 2), Methylcarbamoyl - methyl, Dimethylcarbamoyl - methyl, Phenyl, o - Tolyl, m - Tolyl, p - Fluorphenyl, m - Fluorphenyl, p - Chlorphenyl, p - Tolyl, o - Chlorphenyl, o,p - Dichlorphenyl, o,p - Difluorphenyl, m - Trifluormethylphenyl, o - Fluorphenyl, 3,5 - Dimethylphenyl, 3,5 - Dichlorphenyl, p - Bromphenyl, m - Bromphenyl, 3,5 - Difluorphenyl, Pyrrolidinyl.

$R^2$ und $R^3$ können unabhängig voneinander u.a.bedeuten: Fluor, Chlor, Brom, Jod, Nitro, Methyl, Äthyl, n - Propyl, i - Propyl n - Butyl, i - Butyl, sec.Butyl, tert. - Butyl, n - Pentyl, 2 - Pentyl, 3 - Pentyl, tert. - Amyl, Neopentyl, 2 - Methylbutyl, 3 - Methylbutyl, 3 - Methyl - 2 - butyl, Cyclopentyl, n - Hexyl, 4 - Methyl - 2 - pentyl, 2,3 - Dimethylbutyl, 2 - Methyl - 1 - pentyl, 2 - Hexyl, 3 - Hexyl, 3 - Methyl - 2 - pentyl, 3 - Methylpentyl, 4,4 - Dimethylbutyl, Cyclohexyl, Methoxyäthyl, 3 - Methoxypropyl, Methoxyisopropyl, Chlormethyl, Trichlormethyl, Trifluormethyl, Difluormethyl, Fluormethyl, Jodmethyl, (3′, 4′ - Dichlor) - phenylmethyl, Benzyl, o - Tolyl, p - Fluorphenyl, p - Bromphenyl, m - Chlorphenyl, Cyano, Thiocanato, Methoxycarbonyl, Isopropoxycarbonyl, Äthoxycarbonyl, N - Methyl - N - phenyl - aminocarbonyl, Dimethylaminocarbonyl, Methyläthylaminocarbonyl, Diäthylaminocarbonyl, Diisopropylaminocarbonyl, p - Chlorphenyl - methyl - aminocarbonyl, Dimethylamino, Diäthylamino, Diisopropylamino, Methoxy, Äthoxy, Isopropoxy, Trifluormethoxy, Methylsulfonyl, Phenylsulfonyl, Isopropylsulfonyl, Methoxysulfonyl, Äthoxysulfonyl, Isopropoxysulfonyl, Dimethylaminosulfonyl, Diäthylaminosulfonyl, Piperidinosulfonyl, Methylcarbonyl, Äthylcarbonyl, Isopropylcarbonyl, tert. - Butylcarbonyl, p - Fluorphenylcarbonyl, (4′ - Trifluormethyl - 2′ - nitro) - phenylcarbonyl, 1′,1′,2′,2′ - Tetrafluoräthoxy, 2′,2′,2′ - Trichloräthoxy, 2′ - Chlor - 2′,2′ - difluor - 1′,1′ - difluoräthoxy, Methylthio, Propylthio, Isopropylthio, tert.Butylthio.

Es ist bekannt (DE—OS 26 56 289), Benzothiadiazinonverbindungen der Formel I herzustellen, indem man Verbindungen der allgemeinen Formel II

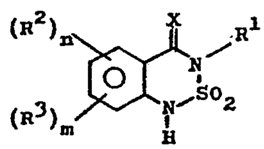

II

in welcher $R^1$, $R^2$, $R^3$, X, m und n die vorgenannten Bedeutungen haben, mit einer Verbindung der allgemeinen Formel III

$$Hal—C≡N, \qquad III$$

wobei Hal Cl oder Br bedeutet, gegebenenfalls in Gegenwart eines Säurebinders und gegebenenfalls in Gegenwart eines inerten Lösungsmittels umsetzt oder indem man die Salze der Verbindungen der Formel II mit Verbindungen der Formel III gegebenenfalls in Gegenwart eines inerten Lösungsmittels umsetzt.

Obwohl dieses Verfahren bei Durchführung im Labormaßstab die gewünschten Produkte in befriedigenden Ausbeuten liefert, treten bei der Übertragung dieser Umsetzung in den technischen Maßstab sehr große Probleme auf.

So können Bromcyan und Chlorcyan wegen der Transportrisiken nicht in größeren Einzelmengen gekauft und transportiert werden.

Ferner verbietet die Instabilität dieser Verbindungen (G. Sorbe, Giftige und explosive Substanzen, S. 46, Umschau-Verlag, Frankfurt/Main, 1968) eine Lagerung der Halogencyane vor der Umsetzung.

Insbesondere müssen bei der Anwendung der Halogencyane in technischem Maßstab wegen der außerordentlichen Giftigkeit dieser Verbindungen strengste Sicherheitsvorkehrungen getroffen werden, die die Durchführung des Verfahrens komplizieren und umständlich machen. Die bei dem bekannten Verfahren verwendeten Basen, soweit sie organischer Natur sind, können mit Halogencyanen unter bestimmten Bedingungen reagieren und unerwünschte Nebenprodukte liefern, so daß die Ausbeuten, insbesondere bei der Umsetzung reaktionsträger Verbindungen der allgemeinen Formel II mit Halogencyanen stark absinken können. Bei der Verwendung von anorganischen Basen als Säurebinder oder von

Salzen der Verbindungen der Formel II liegen die Ausgangsprodukte wegen der Schwerlöslichkeit dieser Verbindungen in den beschriebenen inerten Lösungsmitteln z.T. in fester Form vor, wobei während der Umsetzung noch zusätzlich anorganische Halogenide in fester Form ausfallen, die einerseits die evtl. im inerten Lösungsmittel ebenfalls schwerlöslichen Ausgangsverbindungen einschließen oder umhüllen, so daß diese der Umsetzung entzogen werden, oder die andererseits die Aufarbeitung der Reaktionsmischung und die Abtrennung des Endproduktes erschweren.

Schließlich fallen die gewünschten Endprodukte in den beschriebenen inerten Lösungsmitteln als Lösung an, aus denen sie mit hohem technischen Aufwand und mit viel Energie isoliert werden müssen. Außerdem müssen die organischen Lösungsmittel aus Gründen des Umweltschutzes möglichst quantitativ zurückgewonnen werden.

Es wurde nun überraschenderweise gefunden, daß man alle diese Schwierigkeiten bei der Herstellung von Verbindungen der Formel I vermeidet, wenn man zu einer Mischung von Brom und Wasser vorzugsweise bei einer Temperatur von −5 bis +50°C eine Lösung einer etwa äquimolaren Menge eines anorganischen Cyanids in Wasser zusetzt, danach eine wäßrige Lösung einer höchstens äquimolaren Menge eines Salzes einer Verbindung der Formel II mit einer Base zusetzt, die Mischung gut durchmischt, vorzugsweise bis kein Endprodukt mehr aus der Mischung ausfällt und das entstandene Endprodukt von der Mischung abtrennt.

Vorzugsweise führt man die Umsetzung bei Raumtemperatur (20°C) aus. Man kann aber auch bei einer erhöhten Temperature (20 bis 50°C) arbeiten. Man kann als anorganisches Cyanid Kaliumcyanid oder bevorzugt Natriumcyanid verwenden. Als Salz der Verbindung II mit einer Base kann man das Kaliumsalz oder bevorzugt das Natriumsalz verwenden. Das Endprodukt kann aus den Reaktionsmischung beispielsweise in flüssiger Form mit einem organischen Lösungsmittel, z.B. Dichloräthan, extrahiert oder vorzugsweise in fester Form durch Abfiltrieren, Dekantieren oder Abhebern der Mutterlauge abgetrennt werden. Eine gute Durchmischung der Reaktionsmischung wird beispielsweise durch intensives Rühren der Mischung erzielt.

In einer bevorzugten Ausführungsform tropft man zu einer Emulsion oder Lösung von Brom in Wasser bei einer Temperatur von 0 bis 5°C eine äquimolare Menge einer Lösung eines Alkalicyanids in Wasser bis zur völligen Entfärbung und gibt dann 0,5 bis 1 Mol Äquivalente einer Lösung einer 2,1,3-Benzothiadiazinverbindung der Formel II gelöst in einer äquimolaren Menge verdünnter wäßriger Alkalilauge zu und verfährt dann weiter wie oben angegeben.

Gegebenenfalls kann die bei der bekannten technischen Herstellung der jeweiligen 2,1,3-Benzothiadiazin-4-on-verbindung der Formel II anfallende wäßrig-alkalische Lösung dieser Verbindung nach vorheriger Neutralisation etwa überschüssigen Alkalis mit Säure direkt ohne vorherige Isolierung der 2,1,3-Benzothiadiazinverbindung der Formel II für das erfindungsgemäße Verfahren verwendet werden.

Das erfindungsgemäße Verfahren ist außerordentlich einfach und benötigt zu seiner Durchführung keinerlei organische Lösungsmittel, die nach beendeter Reaktion zurückgewonnen oder vernichtet werden müßten, und deren Verwendung die Anwendung von zusätzlichen sicherheitstechnischen Maßnahmen verlangt. Von besonderem Vorteil ist, daß man von den gut handhabbaren Chemikalien anorganisches Cyanid und Brom ausgeht und die Reaktionspartner in flüssigem Zustand in eine geschlossene Apparatur einbringen kann, so daß ein Kontakt von Personen mit dem äußerst giftigen Bromcyan praktisch ausgeschlossen ist. Durch diese Vermeidung von Lagerung, Transport und Handhabung von Halogencyan ist das erfindungsgemäße Verfahren der bekannten Methode weit überlegen.

Überraschenderweise treten bei dem erfindungsgemäßen Verfahren kaum Nebenreaktion auf, so daß selbst bei der Umsetzung reaktionsträger Verbindungen der allgemeinen Formel II, die längere Reaktionszeiten oder höhere Reaktionstemperaturen benötigt, die Cyanverbindungen der Formel I in sehr guten Ausbeuten entstehen, während bei ihrer Herstellung nach dem bekannten Verfahren die Ausbeuten nur mäßig sind. So kann z.B. 8-Chlor-1-cyano-3-isopropyl-2,1,3-benzothiadiazin-4-on-2,2-dioxid nach dem in der DE—OS 26 56 289 beschriebenen Verfahren in nur ca. 30-prozentiger Ausbeute, nach dem erfindungsgemäßen Verfahren dagegen in ca. 95-prozentiger Ausbeute erhalten werden (vgl. Beispiel 4).

Auch in Bezug auf die Anforderungen des Umweltschutzes bringt das neue Verfahren wesentliche Fortschritte. Wegen der sehr guten Ausbeuten fallen nur geringe Mengen organischer Nebenprodukte an, die gegebenenfalls mit geringen Mengen organischer Lösungsmittel aus der Mutterlauge extrahiert werden können. Das in der wäßrigen Lösung enthaltene Reaktionsprodukt Natriumbromid ist in der Natur weit verbreitet und belastet die Umwelt nicht. In der wäßrigen Lösung enthaltenes unverbrauchtes Bromcyan kann schnell und vollständig durch Zugabe einer gegenüber dem Bromcyan mindestens doppelt molaren Menge einer basischen Verbindung z.B. Natronlauge, Sodalösung oder bevorzugt Ammoniak bzw. eine Lösung von Ammoniak in Wasser zersetzt werden, wobei die Zugabe vor oder nach der Abtrennung des Endproduktes erfolgen kann. Bei der Zersetzung des Bromcyans mit Ammoniak entsteht neben dem umweltneutralen Ammoniumbromid das in Form seiner Salze, als Pflanzennährstoff bekannte Cyanamid. Aus diesem entsteht mit Wasser über die Stufe des Harnstoffs schließlich Ammoniumhydrogencarbonat. Verwendet man zur Zersetzung überschüssigen Bromcyans z.B. Natronlauge, so entsteht neben Natriumbromid Natriumcyanat, das von Wasser sehr schnell zu Ammoniumhydrogencarbonat abgebaut wird. Dieses Salz ist umweltneutral und findet z.B. von Alters her unter dem Namen Hirschhornsalz Verwendung als Bestandteil des Backpulvers im Haushalt.

Wie oben angegeben, fällt die nach dem erfindungsgemäßen Verfahren hergestellte Verbindung der Formel I als Festsubstanz aus wäßriger Lösung an und kann abfiltriert werden. Soll das Endprodukt aber später in einem organischen Lösungsmittel gelöst und in dieser Form weiter verwendet werden oder zur Anwendung kommen, so kann man es natürlich auch mit diesem Lösungsmittel direkt aus der wäßrigen Reaktionsmischung extrahieren. In einem solchen Fall kann man das organische Lösungsmittel zu jedem Zeitpunkt nach der Zugabe des anorganischen Cyanids zusetzen, vorausgesetzt, daß dieses Lösungsmittel nicht mit Bromcyan oder einen anderen Reaktonspartner reagiert.

Bei dem erfindungsgemäßen Verfahren können als anorganische Cyanide alle Cyanide Verwendung finden, die in wäßriger Lösung Cyanidionen bilden, vorzugsweise Cyanide der Metalle der ersten und zweiten Periode des Periodischen Systems der Elemente, insbesondere Natriumcyanid und Kaliumcyanid.

Zur Herstellung der Salze der 2,1,3-Benzothiadiazin-4-on-2,2-dioxide kann man eine Vielzahl von Basen verwenden unter der Voraussetzung, daß, mit Ausnahme der Anionen der 2,1,3-Benzothiadiazin-4-on-2,2-dioxide, sämtliche Neutralisationsprodukte z.B. die entstehenden Kationen oder schwachen Säuren, gegenüber Bromcyan inert sind. Als Basen eignen sich deshalb besonders die Hydroxide, Hydrogencarbonate und Carbonate der Alkali- und der Erdalkalimetalle, insbesondere Natriumhydroxid, Kaliumhydroxid, Calciumhydroxid, Magnesiumhydroxid, Bariumhydroxid, Natriumhydrogencarbonat, Kaliumhydrogencarbonat, Natriumcarbonat, Kaliumcarbonat, Magnesiumcarbonat, Calciumcarbonat, Bariumcarbonat.

Die Umsetzungen können sowohl bei tiefen Temperaturen (beispielsweise —5 bis 19°C) durchgeführt werden, wobei durch die Kristallisation des Wassers eine natürliche untere Grenze gesetzt ist, als auch bei Raumtemperatur (20°C) als auch bei erhöhter Temperatur (beispielsweise 21 bis 50°C), wobei der Höhe der Temperatur durch die Flüchtigkeit des Bromcyans eine natürliche Grenze gesetzt ist. Durch Arbeiten unter erhöhtem Druck (beispielsweise 1 bis 5 bar) läßt sich jedoch auch bei höheren Temperaturen (beispielsweise 50 bis 100°C arbeiten. Insgesamt kann also beispielsweise bei —5 bis +100°C gearbeitet werden.

Die folgenden Beispiele sollen die Durchführung des erfindungsgemäßen Verfahrens und die Vorteile gegenüber dem bekannten Verfahren demonstrieren.

Das erfindungsgemäße Verfahren ist aber nicht auf diese Beispiele beschränkt.

## Beispiel 1

Zu einer gut gerührten Emulsion von 48 Gewichtsteilen Brom in 90 Gewichtsteilen Wasser tropft man bei 0 bis 5°C eine Lösung von 19,5 Gewichtsteilen Kaliumcyanid in 180 Gewichtsteile Wasser. Dazu tropft man eine Lösung von 48 Gewichtsteilen 3-Isopropyl-1H-2,1,3-benzothiadiazin-(4)-3H-on-2,2-dioxid und 18,5 Gewichtsteilen Natriumhydrogencarbonat in 240 Gewichtsteilen Wasser und rührt die Mischung 36 Stunden bei Raumtemperatur (20°C). Der ausgefallene Niederschlag wird abgesaugt, mit Wasser gewaschen und liefert nach dem Trocknen 48,2 Gewichtsteile 1-Cyano-3-isopropyl-1H-2,1,3-benzothiadiazin-(4)-3H-on-2,2-dioxid vom Schmelzpunkt 99 bis 101°C.

## Beispiel 2

Zu einer gut gerührten Emulsion von 4800 Gewichtsteilen Brom in 24 000 Gewichtsteilen Wasser tropft man bei 0 bis 5°C eine Lösung von 1 470 Gewichtsteilen Natriumcyanid in 5 000 Gewichtsteilen Wasser. Dazu gibt man eine Lösung von 800 Gewichtsteilen Natriumhydroxid und 5 080 Gewichtsteilen 3-Iospropyl-8-methyl-1H-2,1,3-benzothiadiazin-(4)-3H-on-2,2-dioxid in 20 000 Gewichtsteilen Wasser hinzu und rührt die Mischung 48 Stunden bei Raumtemperatur (20°C). Danach gibt man 800 Gewichtsteile 25-prozentige Ammoniaklösung zu, rührt nicht länger als 10 Minuten bei Raumtemperatur, saugt den Niederschlag ab und wäscht ihn mit Wasser. Nach dem Trocknen erhält man 5 320 Gewichtsteile 1-Cyano-3-isopropyl-8-methyl-1H-2,1,3-benzothiadiazin-(4)-3H-on-2,2-dioxid vom Schmelzpunkt 104°C.

## Beispiel 3

a) Bekanntes Verfahren (Stand der Technik)

24 Teile 3-Isopropyl-2,1,3-benzothiadiazin-(4)-on-2,2-dioxid werden in 150 Teilen 1,2-Dichloräthan suspendiert und bei 5 bis 10°C mit 12 Teilen Bromcyan versetzt. Danach werden bei dieser Temperatur 14 Teile N,N-Dimethylcyclohexylamin zugetropft und die Mischung 4 Stunden bei Raumtemperatur (20°C) gerührt. Danach wird die Lösung zweimal mit Wasser und zweimal mit kalter 1 normaler wäßriger Natronlauge gewaschen, getrocknet und das Lösungsmittel im Vacuum entfernt. Nach dem Umkristallisieren des Rückstandes aus Isopropanol erhält man 22 Teile 1-Cyano-3-isopropyl-2,1,3-benzothiadiazin-(4)-on-2,2-dioxid vom Schmelzpunkt 100 bis 101°C.

b) Neues Verfahren (erfindungsgemäß)

Zu 17,6 Teilen Brom in 150 Teilen Wasser werden bei 5 bis 10°C 5,4 Teile Natriumcyanid in 40 Teilen Wasser getropft. Danach wird eine Lösung von 24 Teilen 3-Isopropyl-2,1,3-benzothiadiazin-(4)-on-2,2-dioxid und 4 Teilen Natriumhydroxid in 100 Teilen Wasser zugegeben und die Mischung 2 Stunden bei 50°C gerührt. Nach dem Erkalten wird der Niederschlag abgesaugt und getrocknet. Man

erhält 25 Teile 1-Cyano-3-isopropyl-2,1,3-benzothiadiazin-(4)-on-2,2-dioxid vom Schmelzpunkt 99 bis 101°C.

Beispiel 4

a) Bekanntes Verfahren (Stand der Technik)

Zu 274,5 Teilen 8-Chlor-3-isopropyl-2,1,3-benzothiadiazin-(4)-on-2,2-dioxid gelöst in 1 500 Teilen 1,2-Dichloräthan werden bei 5 bis 10°C 127 Teile Bromcyan gegeben und dann bei dieser Temperatur 121 Teile Triäthylamin zugetropft. Anschließend wird 48 Stunden lang bei Raumtemperatur (20°C) nachgerührt und die Reaktionsmischung danach zweimal mit Wasser und dreimal mit 1 normaler wäßriger Natronlauge gewaschen, getrocknet und das Lösungsmittel im Vacuum abgedampft. Der Rückstand wird zweimal aus Isopropanol umkristallisiert und ergibt 90 Teile 8-Chlor-1-cyano-3-isopropyl-2,1,3-benzothiadiazin-(4)-on-2,2-dioxid vom Schmelzpunkt 123 bis 124°C.

b) Neues Verfahren (erfindungsgemäß)

Zu 176 Teilen Brom in 1500 Teilen Wasser wird bei 5 bis 10°C eine Lösung von 54 Teilen Natriumcyanid in 400 Teilen Wasser getropft. Danach wird eine Lösung von 274,5 Teilen 8-Chlor-3-isopropyl-2,1,3-benzothiadiazin-(4)-on-2,2-dioxid und 40 Teilen Natriumhydroxid in 1 000 Teilen Wasser zugegeben und 12 Stunden bei Raumtemperatur (20°C) nachgerührt. Nach dem Absaugen und Trocknen des Niederschlags erhält man 280 Teile 8-Chlor-1-cyano-3-isopropyl-2,1,3-benzothiadiazin-(4)-on-2,2-dioxid vom Schmelzpunkt 123 bis 124°C.

**Patentansprüche**

1. Verfahren zur Herstellung von 1-Cyano-2,1,3-benzothiadiazin-4-on-2,2-dioxiden der allgemeinen Formel I

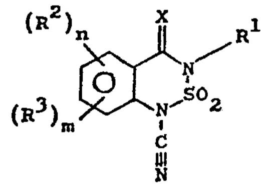

I,

worin

$R^1$ Alkyl, Halogenalkyl, Cycloalkyl, Alkenyl, Alkinyl, Halogenalkenyl, Halogenalkinyl, Alkoxyalkyl, Alkylmercaptoalkyl, Alkyl- und Dialkylcarbamoylalkyl, Alkoxycarbonyl, Alkoxycarboalkyl, Alkoxycarboalkenyl, einen Alkanoylalkyl-, einen gegebenenfalls halogen-, methyl- oder halogen-methylsubstituierten Arylrest oder einen heterocylischen Ring,

$R^2$ und

$R^3$ unabhängig voneinander Halogen, Nitro, Niedrigalkyl, Alkoxyalkyl, Halogenniedrigalkyl, Cycloalkyl, Arylalkyl, Aryl, CN, SCN, $CO_2R^4$, 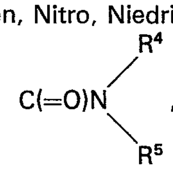 , 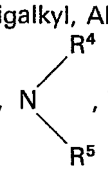 , $YR^5$, $SO_2R^4$, $SO_2OR^4$, 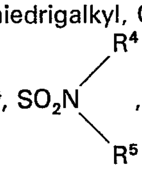 , $(C{=}O)R^4$ oder $Y'CF_2C(Z)_3$,

$R^4$ und

$R^5$ unabhängig voneinander gegebenenfalls halogen-, methyl- oder nitro-substituiertes Niedrigalkyl oder Aryl bedeuten, jedes

X,Y und

Y' unabhängig Sauerstoff oder Schwefel, jedes

Z unabhängig Wasserstoff oder Halogen und

n und

m unabhängig voneinander ganze Zahlen von 0 bis 4 bedeuten mit der Maßgabe, daß m + n nicht größer als 4 ist. *dadurch gekennzeichnet,* daß man zu einer Mischung von Brom und Wasser eine etwa äquimolare Menge eines anorganischen Cyanids, gelöst in Wasser, zusetzt, danach eine wäßrige Lösung einer höchstens äquimolaren Menge eines Salzes aus einem 2,1,3-Benzothiadizin-4-on-2,2-dioxid der Formel II

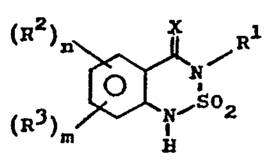

II

in welcher $R^1$, $R^2$, $R^3$, n, m und X die oben angegebene Bedeutung haben, und einer Base zusetzt, die Mischung gut durchmischt und das entstandene Endprodukt von der Mischung abtrennt.

2. Verfahren gemäß Anspruch 1, *dadurch gekennzeichnet,* daß man die Umsetzung bei Raumtemperatur durchführt.

3. Verfahren gemäß Anspruch 1, *dadurch gekennzeichnet,* daß man die Umsetzung bei erhöhter Temperatur durchführt.

4. Verfahren gemäß Anspruch 1, *dadurch gekennzeichnet,* daß man als anorganisches Cyanid Natrium- oder Kaliumcyanid verwendet.

5. Verfahren gemäß Anspruch 1, *dadurch gekennzeichnet,* daß man als Salz der Verbindungen der Formel II das Natrium- oder Kaliumsalz verwendet.

6. Verfahren gemäß Anspruch 1, *dadurch gekennzeichnet,* daß man das Endprodukt von der Mischung abfiltriert oder aus der Mischung mit einem organischen Lösungsmittel extrahiert.

7. Verfahren gemäß Anspruch 1, *dadurch gekennzeichnet,* daß man nach der Umsetzung in der Mischung gegebenenfalls noch vorhandenes Bromcyan vor oder nach der Abtrennung des Endproduktes dadurch entfernt, daß man der Mischung eine gegenüber dem Bromcyan mindestens doppelt molare Menge einer basischen Verbindung zusetzt.

8. Verfahren gemäß Anspruch 1, *dadurch gekennzeichnet,* daß man Ammoniak, Natronlauge oder Sodalösung zusetzt und gegebenenfalls erwärmt.

## Revendications

1. Procédé pour la préparation de 2,2-dioxydes de 1-cyano-2,1,3-benzothiadiazine-4-one de formule générale I

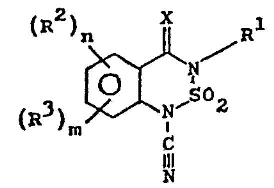

I,

dans laquelle

$R^1$ représente un reste alkyle, halogène-alkyle, cycloalkyle, alcényle, alcynyle, halogène-alcényle, halogène-alcynyle, alcoxyalkyle, alkylmercaptoalkyle, alkyl- et dialkylcarbamoylalkyle, alcoxycarbonyle, alcoxycarboalkyle, alcoxycarboalcényle, alcanoylalkyle, un reste aryle, éventuellement substitué par un halogène, un reste méthyle ou halogènométhyle ou un noyau hétérocyclique.

$R^2$ et $R^3$ représentent indépendamment l'un de l'autre un halogène, un reste nitro, alkyle inférieur, alcoxyalkyl, halogène-alkyle(inférieur), cycloalkyle, arylalkyle, aryle, CN, SCN, $CO_2R^4$, C(=O)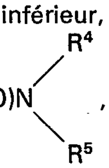 ,

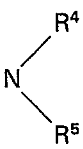 , $YR^5$, $SO_2R^4$, $SO_2OR^4$, $SO_2$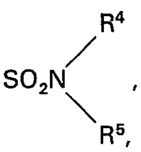 , $(C{=}O)R^4$ ou $Y'CF_2C(Z)_3$,

$R^4$ et $R^5$ représentant chacun indépendamment l'un de l'autre un reste alkyle inférieur ou aryle éventuellement substitué par un halogène, un reste méthyle ou nitro,

X, Y et Y' représentant indépendamment de l'oxygène ou du soufre, chacun

Z représentant indépendamment de l'hydrogène ou un halogène,

n et m représentant indépendamment l'un de l'autre des nombres entiers de 0 à 4, sous réserve que m + n ne soit pas supérieur à 4,

caractérisé par le fait que l'on ajoute à un mélange de brome et d'eau une quantité sensiblement équimolaire d'un cyanure minéral, dissous dans l'eau, que l'on ajoute ensuite une solution aqueuse d'une quantité au plus équimolaire d'un sel d'un 2,2-dioxyde de 2,1,3-benzothiadiazine-4-one de formule II

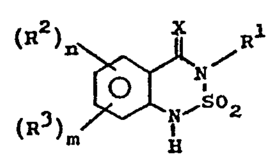

II

dans laquelle $R^1$, $R^2$, $R^3$, n, m et X ont la signification indiquée,

et une base, on brasse énergiquement le mélange et l'on sépare le produit final formé du mélange.

2. Procédé selon la revendication 1, caractérisé par le fait que l'on effectue la réaction à la température ambiante.

3. Procédé selon la revendication 1, caractérisé par le fait que l'on effectue la réaction à température élevée.

4. Procédé selon la revendication 1, caractérisé par le fait que l'on utilise comme cyanure minéral du cyanure de sodium ou de potassium.

5. Procédé selon la revendication 1, caractérisé par le fait que l'on utilise comme sel des composés de formule II le sel de sodium ou de potassium.

6. Procédé selon la revendication 1, caractérisé par le fait que l'on sépare le produit final du mélange par filtration ou qu'on l'extrait du mélange avec un solvant organique.

7. Procédé selon la revendication 1, caractérisé par le fait qu'après la réaction, on élimine le bromure de cyanogène pouvant rester encore dans le mélange, avant ou après la séparation du produit final, en ajoutant au mélange une quantité au moins deux fois molaire, par rapport au bromure de cyanogène, d'un composé basique.

8. Procédé selon la revendication 7, caractérisé par le fait que l'on ajoute de l'ammoniaque, de la lessive de soude ou une solution de soude et qu'on chauffe le cas échéant.

## Claims

1. A process for the preparation of a 1-cyano-2,1,3-benzothiadiazin-4-one-2,2-dioxide of the general formula I

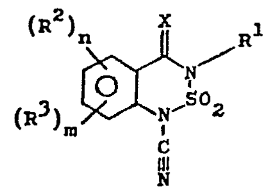

I,

where

$R^1$ is alkyl, haloalkyl, cycloalkyl, alkenyl, alkynyl, haloalkenyl, haloalkynyl, alkoxyalkyl, alkylmercaptoalkyl, alkylcarbamoylalkyl and dialkylcarbamoylalkyl, alkoxycarbonyl, alkoxycarboalkyl, alkoxycarboalkenyl, alkanoylalkyl, aryl (which is unsubstituted or is substituted by halogen, methyl or halomethyl) or a heterocyclic ring,

$R^2$ and $R^3$ independently of one another are halogen, nitro, lower alkyl, alkoxyalkyl, halo-lower alkyl, cycloalkyl, arylalkyl, aryl, CN, SCN, $CO_2R^4$, 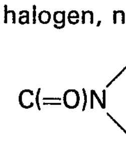 , 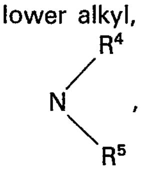 , $YR^5$, $SO_2R^4$, $SO_2OR^4$,

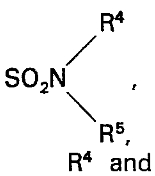 , $(C{=}O)R^4$ or $Y'CF_2C(Z)_3$,

$R^4$ and $R^5$ independently of one another are unsubstituted or halogen-, methyl- or nitro-substituted lower alkyl or aryl,

X, Y and Y' independently of one another are oxygen or sulfur,

each Z, independently of the others, is hydrogen or halogen and

n and m independently of one another are integers from 0 to 4, but m + n is not greater than 4, *characterised in that* an about equimolar amount of an inorganic cyanide, dissolved in water, is added to a mixture of bromine and water, an aqueous solution of an at most equimolar amount of a salt of a 2,1,3-benzothiadiazin-4-one-2,2-dioxide of the formula II

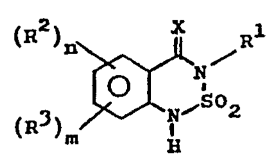

II

where $R^1$, $R^2$, $R^3$, n, m and X have the above meanings, and of a base is then added, the batch is mixed thoroughly and the resulting end product is isolated from the mixture.

2. A process as claimed in claim 1, *characterised in that* the reaction is carried out at room temperature.

3. A process as claimed in claim 1, *characterised in that* the reaction is carried out at elevated temperature.

4. A process as claimed in claim 1, *characterised in that* sodium cyanide or potassium cyanide are used as the inorganic cyanide.

5. A process as claimed in claim 1, *characterised in that* the salt of the compound of the formula II is the sodium salt or the potassium salt.

6. A process as claimed in claim 1, *characterised in that* the end product is filtered off from the mixture or is extracted from the mixture by means of an organic solvent.

7. A process as claimed in claim 1, *characterised in that* any cyanogen bromide which may still be present in the mixture after the reaction is removed, either before or after isolation of the end product, by adding at least two moles, per mole of cyanogen bromide, of a basic compound to the mixture.

8. A process as claimed in claim 7, *characterised in that* ammonia, sodium hydroxide solution or sodium carbonate solution is added and, if desired, the mixture is heated.